# EUROPÄISCHE PATENTSCHRIFT

(11) **EP 3 459 473 B1**
(45) Veröffentlichungstag und Bekanntmachung des Hinweises auf die Patenterteilung: **05.05.2021**
(21) Anmeldenummer: 18194917.3
(22) Anmeldetag: 17.09.2018
(51) Int. Cl.: A61B 17/29, A61B 34/30, A61B 34/00

(54) **INSTRUMENT**
INSTRUMENT
INSTRUMENT

(30) Priorität: 20.09.2017 DE 102017216682
(43) Veröffentlichungstag der Anmeldung: 27.03.2019
(73) Patentinhaber: Richard Wolf GmbH, 75438 Knittlingen (DE)
(72) Erfinder: Wehrheim, Frank, 75015 Bretten (DE)
(74) Vertreter: Patentanwälte Vollmann Hemmer Lindfeld Partnerschaft mbB

(56) Entgegenhaltungen:
- WO-A1-2014/016337
- WO-A2-2016/123139
- DE-A1-102013 220 019
- DE-A1-102014 218 669
- Filip Jelinek: "Steering and Harvesting Technology for Minimally Invasive Biopsy", , 12. Januar 2015 (2015-01-12), Seiten 1-156, XP055544166, Delft (NL) DOI: https://doi.org/10.4233/uuid:18bc7cc6-153b -4ffe-8da1-474f08a212fc ISBN: 978-94-620-3742-7 Gefunden im Internet: URL:https://repository.tudelft.nl/islandor a/object/uuid:18bc7cc6-153b-4ffe-8da1-474f 08a212fc?collection=research [gefunden am 2019-01-17]

## Beschreibung

Die vorliegende Offenbarung betrifft Instrumente, vorzugsweise in Form eines medizinischen Endoskops zum minimalinvasiven Eingriff bei der Diagnose oder chirurgischen Behandlung eines Patienten. Solche Instrumente können allerdings auch als technische Instrumente verwendet werden.

Medizinische Instrumente dieser Art weisen typischerweise am distalen Ende eines länglichen Schafts einen Werkzeugkopf in Form einer Schere, Zange oder Greifers auf, wobei das Werkzeug über entlang des Schafts geführte Steuerseile mittels einer am proximalen Ende des Schafts angeordneten Handhabe betätigt werden kann. Das Instrument kann auch Teil eines robotergesteuerten Systems sein, bei dem die Handhabe durch eine computergesteuerte Motorik ersetzt ist oder diese unterstützt. Das Werkzeug sitzt dabei häufig auf einem Werkzeugkopf, der seinerseits beweglich sein kann, d.h. gegenüber dem Schaft um die Längsachse drehbar oder gegen diese abwinkelbar. Für diese Bewegungen sind oft zusätzliche Steuerseile und mehrere Schaftabschnitte erforderlich.

Die WO 2014/016337 beschreibt beispielsweise ein Instrument mit einer Mehrzahl von Gelenkabschnitten. Auch die US 6,309,403 B1 oder die EP 0 612 496 A1 beschreiben Instrumente mit einer Vielzahl von gegeneinander abwinkelbaren Segmenten. Die Doktorarbeit "Steering and Harvesting Technology for Minimally Invasive Biopsy" von Filip Jelinek (ISBN: 978-94-6203-742-7) beschreibt in Kapitel 6 ebenfalls ein abwinkelbares laparoskopisches Instrument.

Die Bewegungs- und Betätigungskinematik ist auf dem sehr kleinen Bauraum komplex und erfordert eine Vielzahl von gegeneinander beweglichen Komponenten. Der Montageaufwand dieser Vielzahl von gegeneinander beweglichen Komponenten ist entsprechend komplex. Daher sind die bekannten medizinischen Instrumente dieser Art relativ teuer und nicht für die einmalige Verwendung geeignet. Bei mehrmaliger Verwendung kann allerdings die Sterilisierung wegen der Vielzahl von gegeneinander beweglichen Komponenten aufwändig sein.

Die vorliegende Offenbarung stellt ein Instrument bereit, das für die einmalige Verwendung geeignet ist und weniger gegeneinander bewegliche Komponenten aufweist und dabei einen hohen Grad an Bewegungs- und Betätigungsfreiheit für das Werkzeug sicherstellt.

Gemäß der vorliegenden Erfindung weist das Instrument einen an einem distalen Ende eines länglichen Schafts angelenkten Werkzeugkopf und ein Maulteilpaar zum Scheren, Kneifen oder Greifen auf, wobei das Maulteilpaar ein erstes Maulteil und ein zweites Maulteil aufweist. Der Werkzeugkopf ist dabei gegenüber einer Längsachse des Schafts um einen Polarwinkel θ in einer zur Längsachse parallelen Abwinkelebene mittels mindestens zwei aneinander anliegender konvex gekrümmter Wälzflächen definiert abwinkelbar. Das erste Maulteil und das zweite Maulteil sind in einer zur Abwinkelebene im Wesentlichen senkrechten Werkzeugebene unabhängig voneinander gegenüber dem Werkzeugkopf verschwenkbar und definieren jeweils eine im Wesentlichen zylindermantelförmige oder kegelmantelförmige Werkzeugsteuerseilfläche, auf der jeweils zumindest ein Werkzeugsteuerseilabschnitt in Form eines Wendelabschnitts teilweise auf- bzw. abwickelbar ist. Dabei ist die erste Wälzfläche mittels eines in der Abwinkelebene verlaufenden Stegs in einen ersten Abschnitt und einen zweiten Abschnitt aufgeteilt und die zweite Wälzfläche mittels einer in der Abwinkelebene verlaufenden Nut in einen ersten Abschnitt und einen zweiten Abschnitt aufgeteilt. Der Steg greift dabei bei jeder Abwinkelposition (Polarwinkel θ) des Werkzeugkopfs zumindest teilweise in die Nut ein. Damit wird der Werkzeugkopf auf einfache Weise ohne zusätzliche Komponenten senkrecht zur Abwinkelebene fixiert. Auch ein unerwünschtes Verdrehen der Wälzflächen gegeneinander außerhalb der Abwinkelebene wird durch den steten Steg-Nut-Eingriff verhindert. Der Steg definiert zudem scheitelseitig zwei Abwinkelsteuerseilführungen, wobei die Abwinkelsteuerseilführungen symmetrisch bezüglich der Längsachse verlaufen. Beispielsweise kann die Abwinkelsteuerseilführungen eine Rille zur mindestens teilweisen Einlage eines Abwinkelsteuerseils aufweisen. Damit wird im Zusammenspiel mit dem Steg-Nut-Eingriff ein Abknicken und Abrutschen des Abwinkelsteuerseils vom Steg verhindert.

Damit können die Werkzeugsteuerseile knoten- und fittingfrei und ohne Seilrollen geführt werden. Dies reduziert die Komplexität, Bauteilevielfalt und Produktionskosten derart, dass das Instrument zur EinmalVerwendung geeignet ist. Die Werkzeugsteuerseile können wegen der wendelabschnittsförmigen Führung auf der zylindermantel- oder kegelmantelförmigen Werkzeugsteuerseilfläche bevorzugt ein gut gleitendes Kunstfasermaterial wie beispielsweise Aramidfasern, Ultra-Hochmolekulargewichtige PE-Fasern (UHMWPE), Kevlar®, Dyneema®, Vectran® und/oder Carbon-Nanotubes aufweisen.

Optional kann sich eine erste der mindestens zwei Wälzflächen an einem proximalen Endbereich des Werkzeugkopfs und eine zweite der mindestens zwei Wälzflächen an einem distalen Schaftende befinden, wobei die erste Wälzfläche an jedem Wälzpunkt mit der zweiten Wälzfläche in der Abwinkelebene eine größere konvexe Abrollkrümmung hat als die zweite Wälzfläche. Mit "Krümmung" sei hier das betragsmäßige Krümmungsmaß κ=1/r als Kehrwert des Krümmungsradius r des lokalen Schmiegekreises an jedem Punkt entlang einer Kurve gemeint. Ob eine Links- bzw. Rechtskrümmung vorliegt, d.h. das Vorzeichen der Krümmung, sei hierin relativ zum zugehörigen Bauteilkörper über die Attribute "konvex" bzw. "konkav" definiert. Die Abwinkelebene kann relativ zu einer proximalseitigen Handhabe bzw. Robotersteuerung um die Längsachse drehbar oder fix sein. Bei fixer Abwinkelebene kann ggf. das ganze Instrument gedreht werden, um das Werkzeug in eine gewünschte Raumrichtung abwinkeln zu können.

Durch die größere konvexe Krümmung der werkzeugseitigen ersten Wälzfläche auf der weniger konvex gekrümmten schaftseitigen zweiten Wälzfläche kann eine hohe Beweglichkeit des Werkzeugkopfes erreicht werden, ohne mehrere gegeneinander bewegliche Gelenkabschnitte vorzusehen. Der Polarwinkel θ kann dadurch größer als 90° sein und ggf. bis zu 120° und mehr betragen. Außerdem bewegt sich dadurch der Schwerpunkt des Werkzeugkopfes beim Abwinkeln weniger weit von der Längsachse des Schafts weg, sodass eine kompaktere Funktionsweise erreicht wird. Gegenüber einem herkömmlichen Abwinkein über eine feste Abwinkelachse hat das hierein offenbarte Instrument hingegen den Vorteil, dass die Steuerseile nicht zu sehr abgeknickt werden. Ein Krümmungsverhältnis κ₁/κ₂ zwischen erster Abrollkrümmung κ₁ und zweiter Abrollkrümmung κ₂ von 1,05 bis 1,5 hat sich hierbei als vorteilhaft herausgestellt, insbesondere der Bereich um 1,2 von 1,1 bis 1,3.

Die Wälzflächen können im Wesentlichen zylinderförmig mit einer konstanten Abrollkrümmung ausgestaltet sein oder mit veränderlicher Krümmung, wie etwa in der Abwinkelebene elliptisch oder parabelförmig. Die Wälzflächen müssen dabei nicht die gleiche Form haben, wobei es allerdings vorteilhaft ist, wenn die Wälzflächen jeweils bezüglich der Längsachse einen symmetrischen Krümmungsverlauf haben, wenn das Abwinkeln in die eine oder andere Richtung nicht unterschiedlich ablaufen soll.

Optional können die mindestens zwei Wälzflächen formschlüssig ineinander greifen und dazu vorzugsweise ineinandergreifende Verzahnungen aufweisen. Dadurch verrutschen die Wälzflächen in der Abwinkelebene nicht gegeneinander, sodass ein definiertes Abwinkeln sichergestellt ist. Alternativ oder zusätzlich können sie zu diesem Zweck in Reibschluss miteinander stehen. Vorzugsweise haben die Verzahnungen auf den korrespondierenden Wälzflächen das gleiche Modul bzw. die gleiche Zahnradteilung.

Optional kann der Steg in einem Zentralbereich um die Längsachse höher bezüglich der ersten Wälzfläche sein als in lateralen Außenbereichen. Dies ist besonders vorteilhaft, wenn der Steg scheitelseitig ein Abwinkelsteuerseil führt, das dadurch weniger abgeknickt wird, wenn der Werkzeugkopf abgewinkelt wird. Es können somit kostengünstige Zugseile mit Kunststofffasern verwendet werden.

Optional kann der Steg symmetrisch bezüglich der Längsachse in der Abwinkelebene mindestens abschnittsweise eine Stegkrümmung definieren, wobei die Stegkrümmung kleiner ist als die erste Abrollkrümmung. Der Krümmungskreismittelpunkt bzw. die Krümmungskreismittelpunkte können (bei einem Polarwinkel von 0°) dabei versetzt zur Längsachse liegen.

Optional kann der Steg einen zentralen Knick in der Abwinkelebene definieren und/oder mindestens abschnittsweise die Stegkrümmung mit dem Abstand zur Längsachse abnehmen. Der Steg kann also in der Abwinkelebene mittig spitzförmig zulaufen oder abgerundet sein, etwa durch eine Parabel beschreibbar oder zumindest annäherbar.

Optional kann das Instrument ein im Wesentlichen in der Abwinkelebene verlaufendes Abwinkelsteuerseil aufweisen, das einen ersten Abwinkelsteuerseilabschnitt, einen zweiten Abwinkelsteuerseilabschnitt und einen dritten Abwinkelsteuerseilabschnitt aufweist, wobei der erste Abwinkelsteuerseilabschnitt im Wesentlichen gerade auf einer dritten lateralen Seite in Längsrichtung des Schafts vom Schaft zum Werkzeugkopf führt, wobei der zweite Abwinkelsteuerseilabschnitt im Wesentlichen gerade auf einer der dritten lateralen Seite diametral gegenüberliegenden vierten lateralen Seite in Längsrichtung des Schafts vom Schaft zum Werkzeugkopf führt, und sich der dritte Abwinkelsteuerseilabschnitt jeweils am ersten und zweiten Abwinkelsteuerseilabschnitt anschließt und in kraftschlüssiger Verbindung mit dem Werkzeugkopf steht. Dabei kann der dritte Abwinkelsteuerseilabschnitt mäanderförmig durch den Werkzeugkopf geführt sein. Damit wird eine reibschlüssige Kopplung zwischen dem Abwinkelsteuerseil und dem Werkzeugkopf erzielt, sodass das Abwinkelsteuerseil zügelartig mittels Zugkraft den Werkzeugkopf zur dritten lateralen Seite bzw. zur vierten lateralen Seite hin abwinkeln kann. Der dritte Abwinkelsteuerseilabschnitt kann dabei den ersten und zweiten Abwinkelsteuerseilabschnitt verbinden. Das Abwinkelsteuerseil könnte dann zur Reduktion von Komponenten einstückig ausgebildet sein. Alternativ zu einer einstückigen Ausbildung kann das Abwinkelsteuerseil zweiteilig ausgebildet sein. Der dritte Abwinkelsteuerseilabschnitt kann dabei jeweils ein Abwinkelsteuerseilende des ersten und zweiten Abwinkelsteuerseilabschnitts definieren, wobei das jeweilige Abwinkelsteuerseilende durch Reibschluss, Schlingenführung und/oder eine Endverdickung kraftschlüssig mit dem Werkzeugkopf gekoppelt sein kann. Das Abwinkelsteuerseil kann vorzugsweise das gleiche Material aufweisen wie die Werkzeugsteuerseile oder auch anders ausgestaltet sein.

Optional kann die Werkzeugsteuerseilfläche in der Werkzeugebene einen Durchmesser definieren, wobei die Maulteile in einer schlitzförmigen in der Werkzeugebene verlaufenden Werkzeugaufnahme des Werkzeugkopfs über eine gemeinsame senkrecht zur Werkzeugebene verlaufende Werkzeugachse gelagert sind, wobei die schlitzförmige Werkzeugaufnahme in der Längsrichtung tiefer ist als der Durchmesser der Werkzeugsteuerseilflächen. Mit dieser Option werden ebenfalls die Montage und der Aufbau des Werkzeugkopfs vereinfacht.

Erfindungsgemäß weist das Instrument ein erstes Werkzeugsteuerseil für das erste Maulteil und ein zweites Werkzeugsteuerseil für das zweite Maulteil auf, wobei jedes der Werkzeugsteuerseile einen ersten Werkzeugsteuerseilabschnitt, einen zweiten Werkzeugsteuerseilabschnitt und einen dritten Werkzeugsteuerseilabschnitt aufweist, wobei der erste Werkzeugsteuerseilabschnitt im Wesentlichen in der Werkzeugebene und im Wesentlichen gerade auf einer ersten lateralen Seite in Längsrichtung des Schafts vom Schaft zum jeweiligen Maulteil führt, wobei der zweite Werkzeugsteuerseilabschnitt im Wesentlichen in der Werkzeugebene im Wesentlichen gerade auf einer der ersten lateralen Seite diametral gegenüberliegenden zweiten lateralen Seite in Längsrichtung des Schafts vom Schaft zum jeweiligen Maulteil führt, und sich der dritte Werkzeugsteuerseilabschnitt jeweils am ersten und zweiten Werkzeugsteuerseilabschnitt anschließt und in kraftschlüssiger Verbindung mit dem jeweiligen Maulteil steht. Dabei kann der dritte Werkzeugsteuerseilabschnitt mäanderförmig durch das zugehörige Maulteil geführt sein. Damit wird eine reibschlüssige Kopplung zwischen dem Werkzeugsteuerseil und dem zugehörigen Maulteil erzielt, sodass das Werkzeugsteuerseil zügelartig mittels Zugkraft das zugehörige Maulteil zur ersten lateralen Seite bzw. zur zweiten lateralen Seite hin auslenken kann. Der dritte Werkzeugsteuerseilabschnitt kann dabei jeweils den ersten und zweiten Werkzeugsteuerseilabschnitt verbinden. Die Werkzeugsteuerseile könnten dann zur Reduktion von Komponenten jeweils einstückig ausgebildet sein. Alternativ zu einer einstückigen Ausbildung können die Werkzeugsteuerseile jeweils zweiteilig ausgebildet sein. Der dritte Werkzeugsteuerseil kann dabei jeweils ein Werkzeugsteuerseilende des ersten und zweiten Werkzeugsteuerseilabschnitts definieren, wobei das jeweilige Werkzeugsteuerseilende durch Reibschluss, Schlingenführung und/oder eine Endverdickung kraftschlüssig mit dem zugehörigen Maulteil gekoppelt sein kann.

Optional kann der dritte Werkzeugsteuerseilabschnitt jeweils zumindest teilweise in Form eines Wendelabschnitts auf der Werkzeugsteuerseilfläche des jeweiligen Maulteils aufgewickelt sein. Damit wird der Aufbau und die Montage des der Werkzeugsteuerseile und der Maulteile vereinfacht.

Optional kann jedes Maulteil einen von der Werkzeugsteuerseilfläche radial weg erstreckenden Werkzeugabschnitt aufweisen, wobei der auf der Werkzeugsteuerseilfläche verlaufende dritte Werkzeugsteuerseilabschnitt in Umfangsrichtung vor und hinter dem Werkzeugabschnitt in das jeweilige Maulteil und darin schlingenförmig geführt ist. Damit wird eine hohe Auslenkungsamplitude für die Maulteile ermöglicht.

Erfindungsgemäß ist der auf der Werkzeugsteuerseilfläche verlaufende dritte Werkzeugsteuerseilabschnitt in einem lateralen Außenbereich der Werkzeugsteuerseilfläche in das jeweilige Maulteil geführt. Dadurch wird der dritte Werkzeugsteuerseilabschnitt des ersten Maulteils gegenüber dem dritten Werkzeugsteuerseilabschnitt des zweiten Maulteils vom Schaft zu den Maulteilen hin aufgespreizt, wobei die Werkzeugsteuerseile in radialer Richtung übereinander parallel durch den Schaft geführt sein können.

Die Offenbarung ist nachfolgend anhand von in den Zeichnungen dargestellten Ausführungsbeispielen näher erläutert. Es zeigen:
Fig. 1 eine perspektivische Ansicht auf einen distalen Abschnitt eines Ausführungsbeispiels eines Instruments gemäß der vorliegenden Offenbarung;
Fig. 2 eine Seitenansicht auf die Abwinkelebene eines distalen Abschnitts eines Ausführungsbeispiels eines Instruments gemäß der vorliegenden Offenbarung;
Fig. 3a-e fünf Seitenansichten auf die Abwinkelebene eines distalen Abschnitts eines Ausführungsbeispiels eines Instruments gemäß der vorliegenden Offenbarung in jeweils unterschiedlichen Abwinkelpositionen;
Fig. 4 eine Seitenansicht auf die Werkzeugebene eines distalen Abschnitts eines Ausführungsbeispiels eines Instruments gemäß der vorliegenden Offenbarung;
Fig. 5a-f sechs Seitenansichten auf die Werkzeugebene eines distalen Abschnitts eines Ausführungsbeispiels eines Instruments gemäß der vorliegenden Offenbarung in jeweils unterschiedlichen Auslenkungspositionen der Maulteile;
Fig. 6 eine perspektivische Ansicht auf einen Teilschnitt eines distalen Abschnitts eines Ausführungsbeispiels eines Instruments gemäß der vorliegenden Offenbarung;
Fig. 7 eine perspektivische Explosionsansicht auf einen distalen Abschnitt eines Ausführungsbeispiels eines Instruments gemäß der vorliegenden Offenbarung;
Fig. 8 eine perspektivische Ansicht auf ein Maulteil eines Ausführungsbeispiels eines Instruments gemäß der vorliegenden Offenbarung;
Fig.9 eine perspektivische Ansicht auf einen Werkzeugkopf und ein Abwinkelsteuerseil eines Ausführungsbeispiels eines Instruments gemäß der vorliegenden Offenbarung;
Fig. 10 perspektivische Ansichten von zwei Seiten auf ein Maulteil und ein Werkzeugsteuerseil gemäß einer ersten Ausführungsform eines Ausführungsbeispiels eines Instruments gemäß der vorliegenden Offenbarung; und
Fig. 11 perspektivische Ansichten von zwei Seiten auf ein Maulteil und ein Werkzeugsteuerseil gemäß einer zweiten Ausführungsform eines Ausführungsbeispiels eines Instruments gemäß der vorliegenden Offenbarung.

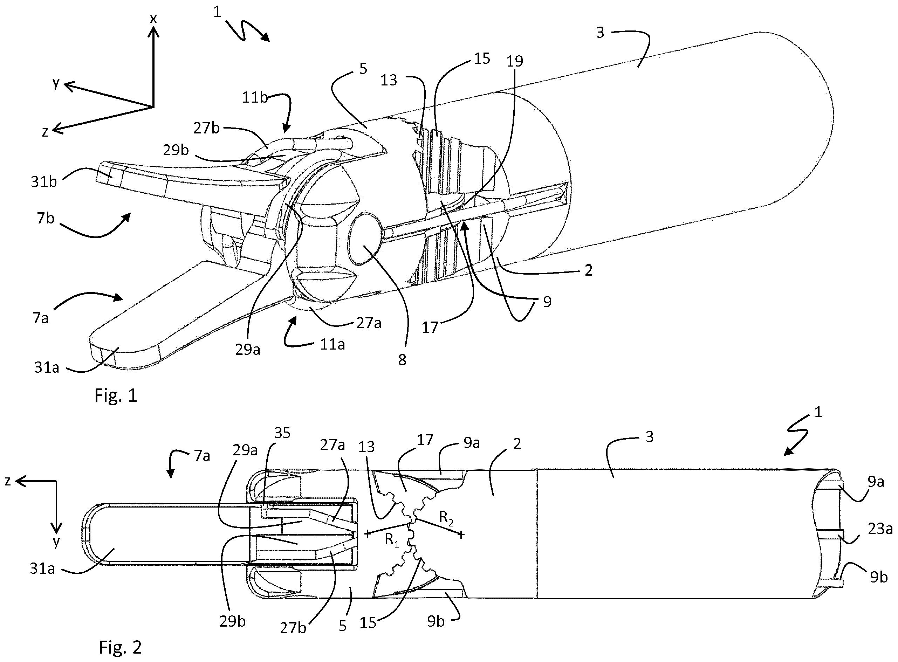

Fig. 1 zeigt in perspektivischer Ansicht einen distalen Abschnitt eines Instruments 1 mit einem an einem distalen Ende 2 eines länglichen Schafts 3 angelenkten Werkzeugkopf 5. Zum Zwecke der einfacheren Erläuterung ist ein rechtshändiges kartesisches Koordinatensystem angegeben, wobei die Längsachse des Schafts 3 in distale Richtung entlang der z-Achse verläuft. Der Werkzeugkopf 5 ist in einer Abwinkelebene y-z gegenüber dem Schaft 3 abwinkelbar, wobei die Abwinkelebene y-z von der y-Achse und der z-Achse aufgespannt wird. Am Werkzeugkopf 5 sind hier zwei Zangenmaulteile 7a,b unabhängig voneinander um eine Werkzeugachse 8 gegenüber dem Werkzeugkopf 5 verschwenkbar, wobei die Werkzeugachse in y-Richtung verläuft. Die x-Achse und die z-Achse spannen somit eine Werkzeugebene x-z auf, in der die zwei Zangenmaulteile 7a,b unabhängig voneinander verschwenkt werden können. "Unabhängig" soll hier bedeuten, dass es keine kontinuierliche Bewegungskopplung zwischen den Zangenmaulteilen 7 gibt. Allerdings kann die Stellung eines Zangenmaulteils 7a,b den Verschwenkungsspielraum des anderen Zangenmaulteils 7b,a einschränken.

Zum Abwinkeln des Werkzeugkopfs 5 gegenüber dem Schaft 3 ist ein Abwinkelsteuerseil 9 vorgesehen, das im Wesentlichen parallel zur Längsachse des Schafts 3 von einer proximalseitigen Handhabe oder Robotersteuerung (nicht gezeigt) zum Werkzeugkopf 5 verläuft. Für das Verschwenken der Zangenmaulteile 7a,b ist für jedes Zangenmaulteil 7a,b jeweils ein Werkzeugsteuerseil 11 a,b vorgesehen, das ebenfalls im Wesentlichen parallel zur Längsachse des Schafts 3 von einer proximalseitigen Handhabe oder Robotersteuerung (nicht gezeigt) zum Werkzeugkopf 5 verläuft.

In der Seitensicht der Fig. 2 auf die Abwinkelebene y-z ist die Abwinkelfunktion besser sichtbar. Proximalseitig am Werkzeugkopf 5 ist eine konvex gekrümmte erste Wälzfläche 13 angeordnet. Die erste Wälzfläche 13 ist hier im Wesentlichen in Form einer Zylindermantelfläche gekrümmt mit einem Krümmungsradius R₁, bzw. einer Abrollkrümmung κ₁=1/R₁. An dem distalen Schaftende 2 eine dazu korrespondierende konvex gekrümmte zweite Wälzfläche 15 auf, wobei sich die erste Wälzfläche 13 beim Abwinkeln des Werkzeugkopfs 5 auf der zweiten Wälzfläche 15 abwälzt. Die zweite Wälzfläche 15 ist hier im Wesentlichen ebenfalls in Form einer Zylindermantelfläche gekrümmt, aber mit einem Krümmungsradius R₂, bzw. einer Abrollkrümmung κ₂=1/R₂. Der Krümmungsradius R₂ der zweiten Wälzfläche 15 ist größer als der Krümmungsradius R₁ der ersten Wälzfläche 13, sodass die erste Wälzfläche 13 an jedem Wälzpunkt mit der zweiten Wälzfläche 15 in der Abwinkelebene y-z eine größere konvexe Abrollkrümmung hat als die zweite Wälzfläche, d.h. κ₁>κ₂.

Um ein Verrutschen der Wälzflächen 13, 15 gegeneinander zu vermeiden und ein definiertes Abwälzen zu ermöglichen, ist sind hier jeweils ineinandergreifende Verzahnungen auf den beiden Wälzflächen 13, 15 vorgesehen. Das Abwinkelsteuerseil 9 weist einen ersten Abwinkelsteuerseilabschnitt 9a auf, der im Wesentlichen gerade auf einer dritten lateralen Seite (die Seite in negative y-Richtung) in Längsrichtung des Schafts 3 vom Schaft 3 zum Werkzeugkopf 5 führt. Außerdem weist das Abwinkelsteuerseil 9 einen zweiten Abwinkelsteuerseilabschnitt 9b auf, der Wesentlichen gerade auf einer der dritten lateralen Seite diametral gegenüberliegenden vierten lateralen Seite (die Seite in positive y-Richtung) in Längsrichtung des Schafts 3 vom Schaft 3 zum Werkzeugkopf 5 führt. Bei Zug am ersten Abwinkelsteuerseilabschnitt 9a winkelt der Werkzeugkopf 5 in negative y-Richtung ab, und bei Zug am zweiten Abwinkelsteuerseilabschnitt 9b winkelt der Werkzeugkopf 5 in positive y-Richtung ab. Solche Zugsteuerbefehle können durch eine mit dem Abwinkelsteuerseil 9 verbundene Handhabe an einem proximalen Ende des Schafts 3 manuell oder durch ein an einem proximalen Ende des Schafts 3 angeschlossenes Robotersteuersystem automatisiert gegeben werden. Eine Robotersteuerung ist hier bevorzugt, da eine Abwinklungs- und Werkzeugsteuerung komplex sein kann.

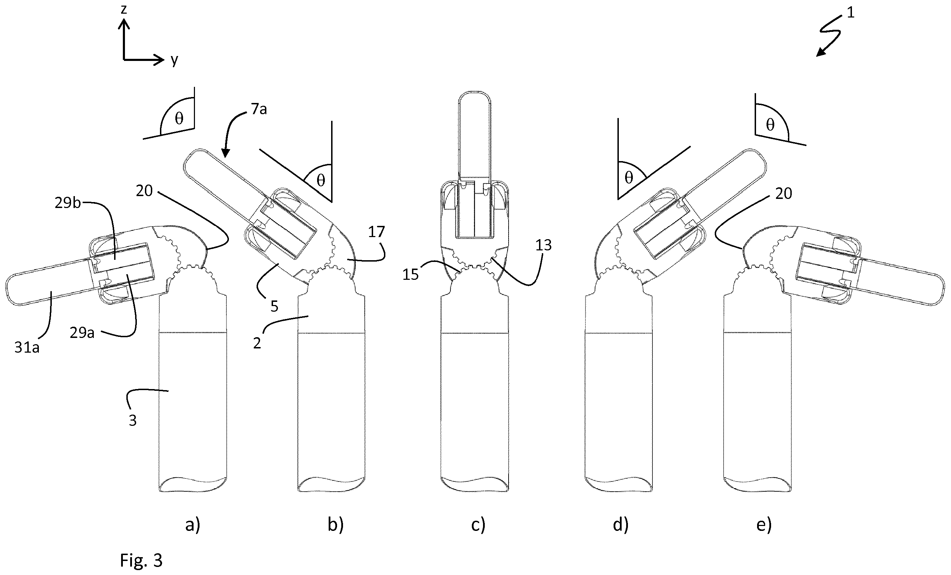

In Figur 3 sind verschiedene Abwinkelpositionen des Instruments 1 um verschiedene Polarwinkel θ ohne das Abwinkelsteuerseil 9 und ohne Werkzeugsteuerseile 11 a,b gezeigt. Der Polarwinkel θ, wobei hier 0°≤θ≤120°, spannt sich zwischen der in z-Richtung verlaufenden Längsachse des Schafts 3 und der Längsachse des Werkzeugkopfs 5 in der Abwinkelebene y-z auf. In Figur 3c ist der Werkzeugkopf 5 nicht abgewinkelt, d.h. θ=0°. In Figuren 3b und 3d ist der Werkzeugkopfs 5 jeweils einmal in die negative y-Richtung und einmal in die positive y-Richtung um 0°<θ<90° abgewinkelt. In Figuren 3a und 3e ist der Werkzeugkopfs 5 jeweils einmal in die negative y-Richtung und einmal in die positive y-Richtung um 90°<θ≤120° abgewinkelt. Dadurch, dass der Krümmungsradius R₂ der zweiten Wälzfläche 15 größer ist als der Krümmungsradius R₁ der ersten Wälzfläche 13 überstreicht jeder Punkt des Werkzeugkopfs einen Teil einer Epizykloide, die keine Kardioide ist. Damit wird ein schnelles und symmetrisches Abwinkeln auf möglichst kleinem Raum bei gleichzeitig möglichst großem Abwinkelbereich bzw. Polarwinkel θ ermöglicht.

Ein in Figur 1 zum Teil sichtbarer Steg 17 des Werkzeugkopfs 5 ist den Seitenansichten von Figur 3 gut erkennbar. Der Steg 17 verläuft in der Abwinkelebene y-z und teilt die erste Wälzfläche 13 in einen ersten Abschnitt und einen zweiten Abschnitt auf. Analog ist die zweite Wälzfläche 15 mittels einer in der Abwinkelebene y-z verlaufenden Nut 19 (siehe Fig. 1) in einen ersten Abschnitt und einen zweiten Abschnitt aufgeteilt. Der Steg 17 greift bei jeder Abwinkelposition bzw. jedem Polarwinkel θ des Werkzeugkopfs 5 zumindest teilweise in die Nut 19 ein und stabilisiert bzw. führt somit den Werkzeugkopf 5 während des Abwinkelns in der Abwinkelebene y-z. Der Steg 17 ist hier in einem Zentralbereich um die Längsachse des Werkzeugkopfs 5 höher bezüglich der ersten Wälzfläche 13 als in lateralen Außenbereichen. Dadurch kann der Steg 19 zum einen bei jeder möglichen Abwinkelstellung bzw. jedem möglichen Polarwinkel θ Eingriff in die Nut 19 halten und zum anderen möglichst große Polarwinkel θ erlauben. Hier läuft der Steg 17 mittig leicht spitz mit einem Knick 20 zu wie in Figuren 3a und 3e zu erkennen ist.

Der Steg 17 definiert scheitelseitig zwei Abwinkelsteuerseilführungen, wobei die Abwinkelsteuerseilführungen symmetrisch bezüglich der Längsachse verlaufen. Die Abwinkelsteuerseilführungen weisen jeweils eine Rille 22 zur mindestens teilweisen Einlage des Abwinkelsteuerseils 9 auf. Damit wird das Abwinkelsteuerseil 9 sicher und ohne Abknicken scheitelseitig auf dem Steg 17 geführt.

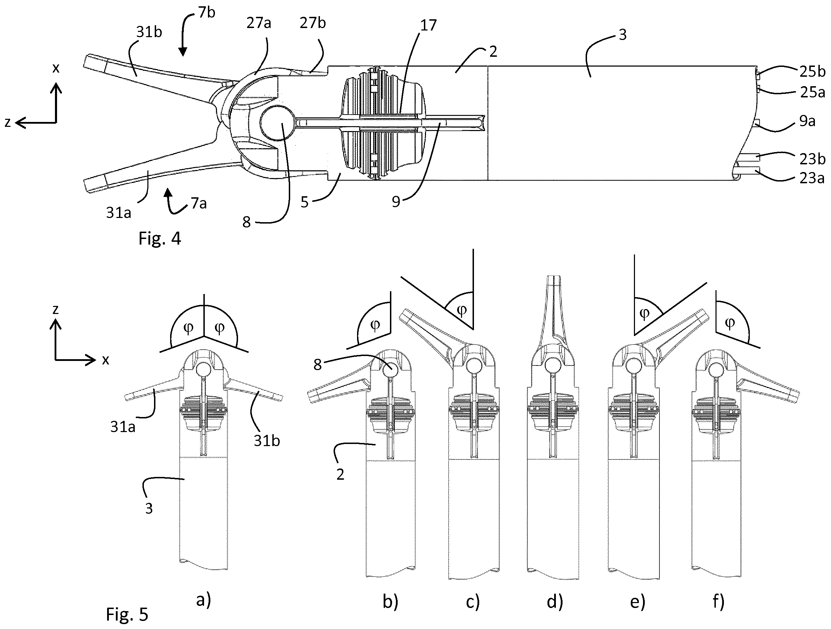

In den Seitenansichten der Figuren 4 und 5 auf die Werkzeugebene x-z ist die Werkzeugmechanik gut erkennbar. Jedes der Zangengenmaulteile 7a,b ist zwischen einem lateralen Anschlagpunkt am Werkzeugkopf 5 und dem Anschlag an das jeweils andere Zangenmaulteil 7a,b unabhängig voneinander um die in y-Richtung verlaufende Werkzeugachse 8 in der Werkzeugebene x-z gegenüber dem Werkzeugkopf 5 verschwenkbar. Hier sind die vom Werkzeugkopf 5 definierten lateralen Anschlagpunkte so weit weg von der Werkzeugachse 8 angeordnet, dass Verschwenkungswinkel ϕ von mehr als 90° und bis zu 120° möglich sind.

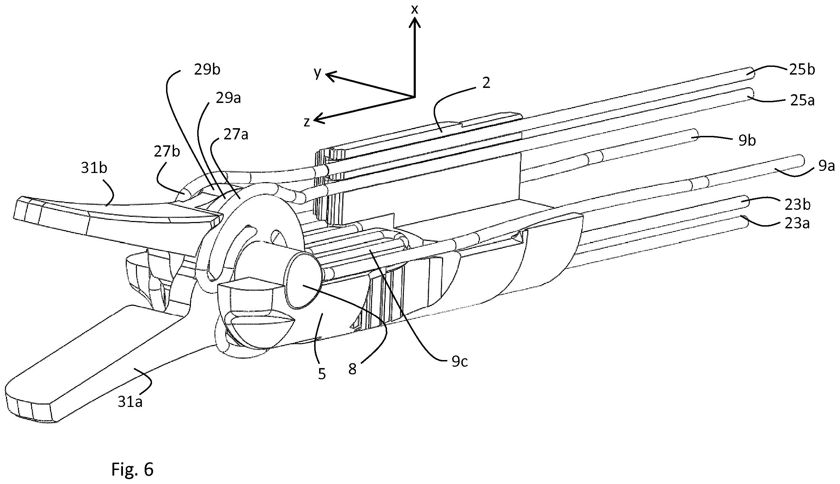

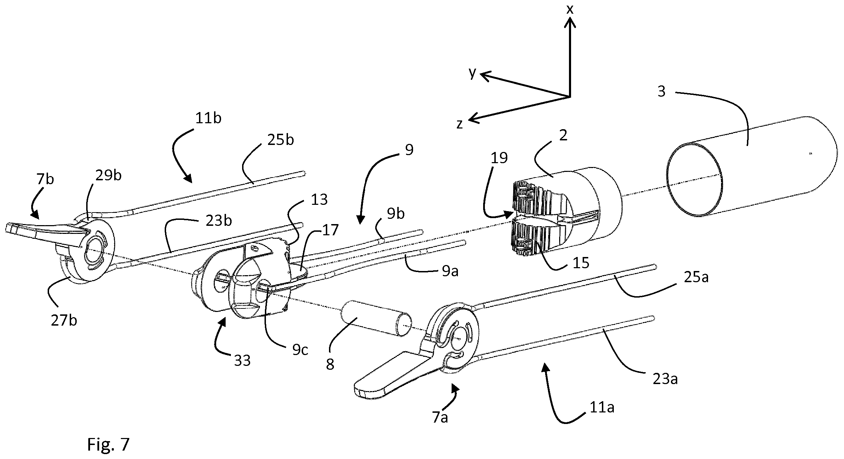

In dem perspektivischen Teilschnitt von Fig. 6 und der Explosionsdarstellung von Fig. 7 wird deutlich wie die einzelnen Zangenmaulteile 7a,b über jeweils ein Werkzeugsteuerseil 11a,b zum Verschwenken betätigt werden. Die Werkzeugsteuerseile 11a,b weisen jeweils einen ersten Werkzeugsteuerseilabschnitt 23a,b, einen zweiten Werkzeugsteuerseilabschnitt 25a,b und einen dritten Werkzeugsteuerseilabschnitt 27a,b auf, wobei der erste Werkzeugsteuerseilabschnitt 23a,b im Wesentlichen in der Werkzeugebene x-z und im Wesentlichen gerade auf einer ersten lateralen Seite (die Seite in negative x-Richtung) in Längsrichtung des Schafts 3 vom Schaft 3 zum jeweiligen Maulteil 7a,b führt, wobei der zweite Werkzeugsteuerseilabschnitt 25a,b im Wesentlichen in der Werkzeugebene x-z im Wesentlichen gerade auf einer der ersten lateralen Seite diametral gegenüberliegenden zweiten lateralen Seite (die Seite in positive x-Richtung) in Längsrichtung des Schafts 3 vom Schaft 3 zum jeweiligen Maulteil 7a,b führt, und sich der dritte Werkzeugsteuerseilabschnitt 27a,b jeweils am ersten 23a,b und zweiten Werkzeugsteuerseilabschnitt 25a,b anschließt und in kraftschlüssiger Verbindung mit dem jeweiligen Maulteil 7a,b steht. Der dritte Werkzeugsteuerseilabschnitt 27a,b verbindet hier den erste Werkzeugsteuerseilabschnitt 23a,b mit dem zweiten Werkzeugsteuerseilabschnitt 25a,b, sodass das Werkzeugsteuerseil 11a,b einstückig ausgebildet ist. Der dritte Werkzeugsteuerseilabschnitt 27a,b verläuft schlingenförmig durch das jeweilige Zangenmaulteil 7a,b, um die kraftschlüssige Verbindung sicherzustellen.

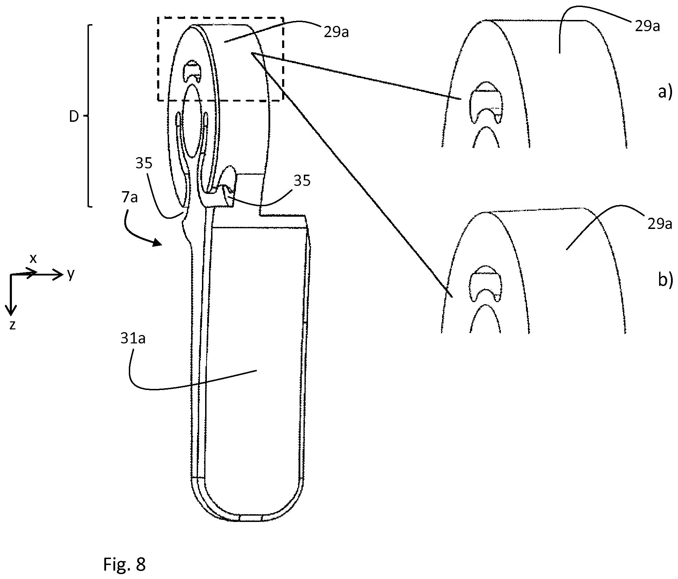

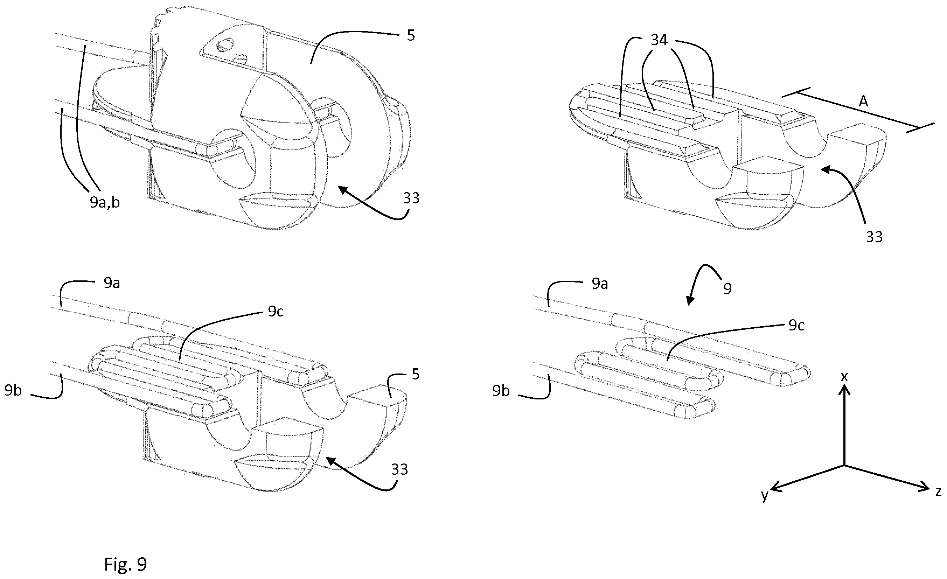

Der dritte Werkzeugsteuerseilabschnitt 27a,b, ist hierbei zumindest teilweise in Form eines Wendelabschnitts auf einer Werkzeugsteuerseilfläche 29a,b, des jeweiligen Maulteils 7a,b aufgewickelt. Die Maulteile 7a,b definieren hier jeweils die im Wesentlichen zylindermantel- oder kegelmantelförmige Werkzeugsteuerseilfläche 29a,b. Jedes Maulteil 7a,b weist dabei einen sich von der Werkzeugsteuerseilfläche 29a,b radial weg erstreckenden Werkzeugabschnitt 31a,b auf, wobei der auf der Werkzeugsteuerseilfläche 29a,b verlaufende dritte Werkzeugsteuerseilabschnitt 27a,b in Umfangsrichtung vor und hinter dem Werkzeugabschnitt 31a,b in das jeweilige Maulteil 7a,b und darin schlingenförmig um die Werkzeugachse 8 geführt ist. Die Werkzeugsteuerseilfläche 29a,b definiert in der Werkzeugebene x-z einen Durchmesser D (siehe Figur 8). Die Maulteile 7a,b verlaufen hier in einer schlitzförmigen in der Werkzeugebene verlaufenden Werkzeugaufnahme 33 des Werkzeugkopfs 5, wobei die schlitzförmige Werkzeugaufnahme 33 in Längsrichtung des Schafts 3 (z-Richtung) tiefer ist als der Durchmesser D der Werkzeugsteuerseilflächen 29a,b. In Fig. 9 ist die Werkzeugaufnahme 33 des Werkzeugkopfs 5 zur Aufnahme der Maulteile 7a,b besser zu sehen, wobei A die Tiefe der schlitzförmigen Werkzeugaufnahme 33 bezeichnet

In Fig. 6 ist gut zu erkennen, dass die ersten Werkzeugsteuerseilabschnitte 23a,b und zweiten Werkzeugsteuerseilabschnitte 25a,b jeweils in radialer Richtung übereinander parallel durch den Schaft 3 geführt sind. Der auf der zugehörigen Werkzeugsteuerseilfläche 29a,b jeweils wendelabschnittsförmig verlaufende dritte Werkzeugsteuerseilabschnitt 27a,b ist in einem lateralen Außenbereich der Werkzeugsteuerseilfläche 29a,b in das jeweilige Maulteil 7a,b geführt. Dadurch wird der dritte Werkzeugsteuerseilabschnitt 27a des ersten Maulteils 7a gegenüber dem dritten Werkzeugsteuerseilabschnitt 27b des zweiten Maulteils über dem dritten Werkzeugsteuerseilabschnitt 27b des zweiten Maulteils 7b vom Schaft 3 zu den Werkzeugabschnitten 31a,b der Maulteile 7a,b hin aufgespreizt. Die Aufspreizung ist besser ist Fig. 2 zu sehen.

In Figur 6 sowie in Figur 9 ist ebenfalls deutlich zu erkennen wie das Abwinkelsteuerseil 9 mit dem ersten Abwinkelsteuerseilabschnitt 9a auf der dritten lateralen Seite (die Seite in negative y-Richtung) in Längsrichtung des Schafts 3 zum Werkzeugkopf 5 führt und mit dem zweiten Abwinkelsteuerseilabschnitt 9b auf der vierten lateralen Seite (die Seite in positive y-Richtung) in Längsrichtung des Schafts 3 zum Werkzeugkopf 5 führt. Zwischen dem ersten Abwinkelsteuerseilabschnitt 9a und dem zweiten Abwinkelsteuerseilabschnitt 9b verläuft ein dritter Abwinkelsteuerseilabschnitt 9c des Abwinkelsteuerseils 9 mäanderförmig durch den Werkzeugkopf 5, um das Abwinkelsteuerseil 9 mit dem Werkzeugkopf 5 in kraftschlüssige Verbindung zu bringen (siehe Figur 9). Das Abwinkelsteuerseil 9 ist hier einstückig ausgebildet. Der Werkzeugkopf 5 weist zur Aufnahme des mäanderförmig durchgeführten dritten Abwinkelsteuerseilabschnitts 9c des Abwinkelsteuerseils 9 eine entsprechend mäanderförmige Schlingenführung 34 auf.

In Fig. 8 ist ein Zangenmaulteil 7a detaillierter dargestellt mit zwei verschiedenen Ausführungsformen einer Werkzeugsteuerseilfläche 29a, die jeweils als Detailansichten 8a) und 8b) dargestellt sind. In Fig. 8a) ist die Werkzeugsteuerseilfläche 29a zylindermantelförmig und in Fig. 8b) ist die Werkzeugsteuerseilfläche 29a kegelmantelförmig. Auf dieser Werkzeugsteuerseilfläche 29a ist zumindest teilweise das Werkzeugsteuerseil 11a in Form eines Wendelabschnitts aufwickelbar. Außerdem sind in Umfangsrichtung vor und hinter dem Werkzeugabschnitt 31a,b angeordnete Öffnungen 35 gezeigt, durch die das Werkzeugsteuerseil 11a in das Zangenmaulteil 7a schlingenförmig führbar ist. Die Öffnungen 35 sind hier in einem lateralen Außenbereich der Werkzeugsteuerseilfläche 29a angeordnet, um die Werkzeugsteuerseile 11a,b aufzuspreizen. Analog ist Fig. 8 für das zweite Zangenmaulteil 7b zu verstehen.

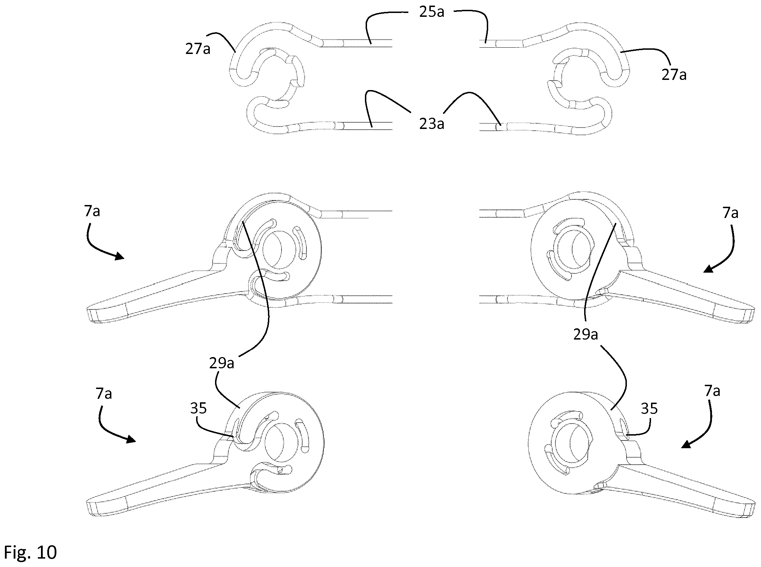

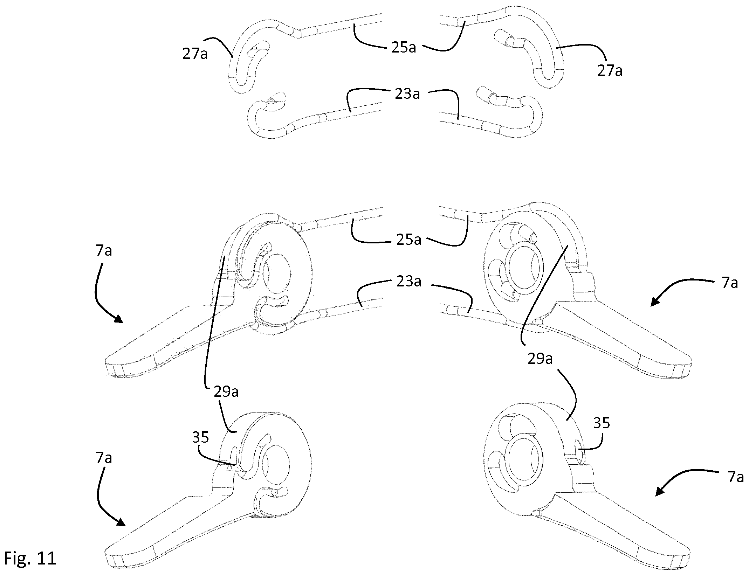

In Fig. 10 ist die schlingenförmige Durchführung eines einstückigen Werkzeugsteuerseils 11a durch das Zangenmaulteil 7a gezeigt. Das Werkzeugsteuerseil 11a ist hier in das Zangenmaulteil 7a "eingenäht" und verläuft in einer Schlaufe mäanderförmig um die mittige Durchbrechung für die Werkzeugachse 8. In Fig. 11 ist eine Ausführungsform gezeigt, bei der das Werkzeugsteuerseil 11a zweistückig ausgebildet ist. Der dritte Werkzeugsteuerseilabschnitt 27a definiert hier jeweils ein distalseitiges Ende des ersten Werkzeugsteuerseilabschnitts 23a bzw. des des zweiten Werkzeugsteuerseilabschnitts 25a, die hier jeweils eine Endverdickung 37a aufweist. Die Endverdickung 37a verhindert ein Durchrutschen des Werkzeugsteuerseils 11a bei dessen Betätigung und stellt somit eine kraftschlüssige Verbindung sicher. Die Figuren 10 und 11 gelten analog für das zweite Maulteil 7b und das zugehörige zweite Werkzeugsteuerseil 11b.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Im Übrigen soll hierin weder der Begriff "aufweisen" zusätzliche andere Merkmale oder Verfahrensschritte ausschließen noch soll "ein" oder "eine" eine Mehrzahl ausschließen.

## Patentansprüche

1. Instrument (1) mit
- einem an einem distalen Ende (2) eines länglichen Schafts (3) angelenkten Werkzeugkopf (5),
- einem Maulteilpaar (7a,b) zum Scheren, Kneifen oder Greifen, wobei das Maulteilpaar (7a,b) ein erstes Maulteil (7a) und ein zweites Maulteil (7b) aufweist, und
- einem ersten Werkzeugsteuerseil (11a) für das erste Maulteil (7a) und einem zweiten Werkzeugsteuerseil (11b) für das zweite Maulteil (7b),
wobei der Werkzeugkopf (5) gegenüber einer Längsachse des Schafts (3) um einen Polarwinkel (θ) in einer zur Längsachse parallelen Abwinkelebene (y-z) mittels mindestens zwei aneinander anliegender konvex gekrümmter Wälzflächen (13, 15) definiert abwinkelbarist,
wobei das erste Maulteil (7a) und das zweite Maulteil (7b) in einer zur Abwinkelebene (y-z) im Wesentlichen senkrechten Werkzeugebene (x-z) unabhängig voneinander gegenüber dem Werkzeugkopf (5) verschwenkbar sind, und
wobei das erste Maulteil (7a) und das zweite Maulteil (7b) jeweils eine im Wesentlichen zylindermantelförmige oder kegelmantelförmige Werkzeugsteuerseilfläche (29a,b) definieren, auf der jeweils zumindest ein Werkzeugsteuerseilabschnitt (27a,b) in Form eines Wendelabschnitts teilweise auf- oder abwickelbar ist,
wobei eine erste Wälzfläche (13) der mindestens zwei Wälzflächen (13, 15) mittels eines in der Abwinkelebene (y-z) verlaufenden Stegs (17) in einen ersten Abschnitt und einen zweiten Abschnitt aufgeteilt ist, und eine zweite Wälzfläche (15) der mindestens zwei Wälzflächen (13, 15) mittels einer in der Abwinkelebene (y-z) verlaufenden Nut (19) in einen ersten Abschnitt und einen zweiten Abschnitt aufgeteilt ist, wobei der Steg (17) bei jeder Abwinkelposition des Werkzeugkopfs (5) zumindest teilweise in die Nut (19) eingreift,
wobei der Steg (17) scheitelseitig zwei Abwinkelsteuerseilführungen definiert,
wobei die Abwinkelsteuerseilführungen symmetrisch bezüglich der Längsachse verlaufen,
wobei jedes der Werkzeugsteuerseile (11a,b) einen ersten Werkzeugsteuerseilabschnitt (23a,b), einen zweiten Werkzeugsteuerseilabschnitt (25a,b) und einen dritten Werkzeugsteuerseilabschnitt (27a,b) aufweist, wobei der erste Werkzeugsteuerseilabschnitt (23a,b) im Wesentlichen in der Werkzeugebene (x-z) und im Wesentlichen gerade auf einer ersten lateralen Seite in Längsrichtung des Schafts (3) vom Schaft zum jeweiligen Maulteil (7a,b) führt, wobei der zweite Werkzeugsteuerseilabschnitt (25a,b) im Wesentlichen in der Werkzeugebene (x-z) im Wesentlichen gerade auf einer der ersten lateralen Seite diametral gegenüberliegenden zweiten lateralen Seite in Längsrichtung des Schafts (3) vom Schaft (3) zum jeweiligen Maulteil (7a,b) führt, und sich der dritte Werkzeugsteuerseilabschnitt (27a,b) jeweils am ersten und zweiten Werkzeugsteuerseilabschnitt (25a,b, 27a,b) anschließt und in kraftschlüssiger Verbindung mit dem jeweiligen Maulteil (7a,b) steht, wobei der dritte Werkzeugsteuerseilabschnitt (27a) des ersten Maulteils (7a) gegenüber dem dritten Werkzeugsteuerseilabschnitt (27b) des zweiten Maulteils (7b) vom Schaft (3) zu den Maulteilen (7a,b) hin in der Abwinkelebene (y-z) aufgespreizt ist, wobei der jeweils auf der Werkzeugsteuerseilfläche (29a,b) verlaufende dritte Werkzeugsteuerseilabschnitt (27a,b) in einem lateralen Außenbereich der Werkzeugsteuerseilfläche (29a,b) in das jeweilige Maulteil (7a,b) geführt ist.

2. Instrument (1) nach Anspruch 1, wobei sich eine erste (13) der mindestens zwei Wälzflächen (13, 15) an einem proximalen Endbereich des Werkzeugkopfs (5) befindet und eine zweite (15) der mindestens zwei Wälzflächen (13, 15) an dem distalen Schaftende (2) befindet, wobei die erste Wälzfläche (13) an jedem Wälzpunkt mit der zweiten Wälzfläche (15) in der Abwinkelebene (y-z) eine größere konvexe Abrollkrümmung hat als die zweite Wälzfläche (15).

3. Instrument (1) nach Anspruch 2, wobei die mindestens zwei Wälzflächen (13, 15) ineinandergreifende Verzahnungen aufweisen.

4. Instrument (1) nach einem der vorhergehenden Ansprüche, wobei der Steg (17) in einem Zentralbereich um die Längsachse höher bezüglich der ersten Wälzfläche (13) ist als in lateralen Außenbereichen.

5. Instrument (1) nach einem der vorhergehenden Ansprüche, wobei der Steg (17) symmetrisch bezüglich der Längsachse in der Abwinkelebene (y-z) mindestens abschnittsweise eine Stegkrümmung definiert, wobei die Stegkrümmung kleiner ist als die erste Abrollkrümmung.

6. Instrument (1) nach einem der vorhergehenden Ansprüche, wobei der Steg (17) einen zentralen Knick (20) in der Abwinkelebene (y-z) definiert und/oder mindestens abschnittsweise die Stegkrümmung mit dem Abstand zur Längsachse abnimmt.

7. Instrument (1) nach einem der vorhergehenden Ansprüche, wobei die Abwinkelsteuerseilführungen eine Rille (22) zur mindestens teilweisen Einlage eines Abwinkelsteuerseils (9) aufweist.

8. Instrument (1) nach einem der vorhergehenden Ansprüche, mit einem im Wesentlichen in der Abwinkelebene (y-z) verlaufenden Abwinkelsteuerseil (9), das einen ersten Abwinkelsteuerseilabschnitt (9a), einen zweiten Abwinkelsteuerseilabschnitt (9b) und einen dritten Abwinkelsteuerseilabschnitt (9c) aufweist, wobei der erste Abwinkelsteuerseilabschnitt (9a) im Wesentlichen gerade auf einer dritten lateralen Seite in Längsrichtung des Schafts (3) vom Schaft (3) zum Werkzeugkopf (5) führt, wobei der zweite Abwinkelsteuerseilabschnitt (9b) im Wesentlichen gerade auf einer der dritten lateralen Seite diametral gegenüberliegenden vierten lateralen Seite in Längsrichtung des Schafts (3) vom Schaft (3) zum Werkzeugkopf (5) führt, und sich der dritte Abwinkelsteuerseilabschnitt (9c) jeweils am ersten (9a) und zweiten Abwinkelsteuerseilabschnitt (9b) anschließt und in kraftschlüssiger Verbindung mit dem Werkzeugkopf (5) steht.

9. Instrument (1) nach Anspruch 8, wobei der dritte Abwinkelsteuerseilabschnitt (9c) mäanderförmig durch den Werkzeugkopf (5) geführt ist.

10. Instrument (1) nach Anspruch 8 oder 9, wobei der dritte Abwinkelsteuerseilabschnitt den ersten und zweiten Abwinkelsteuerseilabschnitt verbindet.

11. Instrument (1) nach Anspruch 8, 9 oder 10, wobei der dritte Abwinkelsteuerseilabschnitt (9c) jeweils ein Abwinkelsteuerseilende des ersten (9a) und zweiten Abwinkelsteuerseilabschnitts (9b) definiert, wobei das jeweilige Abwinkelsteuerseilende durch Reibschluss, Schlingenführung und/oder eine Endverdickung kraftschlüssig mit dem Werkzeugkopf gekoppelt ist.

12. Instrument (1) nach einem der vorhergehenden Ansprüche, wobei die Werkzeugsteuerseilfläche (29a,b) in der Werkzeugebene (x-z) einen Durchmesser (D) definiert, und wobei die Maulteile (7a,b) in einer schlitzförmigen in der Werkzeugebene (x-z) verlaufenden Werkzeugaufnahme (33) des Werkzeugkopfs (5) über eine gemeinsame senkrecht zur Werkzeugebene (x-z) verlaufende Werkzeugachse (8) gelagert sind, wobei die schlitzförmige Werkzeugaufnahme (33) in der Längsrichtung tiefer ist als der Durchmesser (D) der Werkzeugsteuerseilflächen (29a,b).

13. Instrument (1) nach einem der vorhergehenden Ansprüche, wobei der dritte Werkzeugsteuerseilabschnitt (27a,b) mäanderförmig durch das jeweilige Maulteil (7a,b) geführt ist.

14. Instrument (1) nach einem der vorhergehenden Ansprüche, wobei der dritte Werkzeugsteuerseilabschnitt (27a,b) den ersten (23a,b) und zweiten Werkzeugsteuerseilabschnitt (25a,b) verbindet.

15. Instrument (1) nach einem der vorhergehenden Ansprüche, wobei der dritte Werkzeugsteuerseilabschnitt (27a,b) jeweils ein Werkzeugsteuerseilende des ersten (23a,b) und zweiten Werkzeugsteuerseilabschnitts (25a,b) definiert, wobei das jeweilige Werkzeugsteuerseilende durch Reibschluss, Schlingenführung und/oder eine Endverdickung kraftschlüssig mit dem jeweiligen Maulteil (7a,b) gekoppelt ist.

16. Instrument (1) nach einem der vorhergehenden Ansprüche, wobei der dritte Werkzeugsteuerseilabschnitt (27a,b) zumindest teilweise in Form eines Wendelabschnitts auf der Werkzeugsteuerseilfläche (29a,b) des jeweiligen Maulteils (7a,b) aufgewickelt ist.

17. Instrument (1) nach einem der vorhergehenden Ansprüche, wobei jedes Maulteil (7a,b) einen sich von der Werkzeugsteuerseilfläche (29a,b) radial weg erstreckenden Werkzeugabschnitt (31a,b) aufweist, wobei der auf der Werkzeugsteuerseilfläche (29a,b) verlaufende dritte Werkzeugsteuerseilabschnitt (27a,b) in Umfangsrichtung vor und hinter dem Werkzeugabschnitt (31a,b) in das jeweilige Maulteil (7a,b) und darin schlingenförmig geführt ist.

## Claims

1. An instrument (1) with
- a tool head (5) which is articulated on a distal end (2) of an elongate shank (3),
- a jaw part pair (7a, 7b) for shear cutting, pinching or gripping,
wherein the jaw part pair (7a,b) comprises a first jaw part (7a) and a second jaw part (7b), and
- a first tool control cable (11a) for the first jaw part (7a) and a second tool control cable (11b) for the second jaw part (7b);
wherein the tool head (5) can be bent in a defined manner with respect to a longitudinal axis of the shank (3) about a polar angle (θ) in a bending plane (y-z) which is parallel to the longitudinal axis, by way of at least two convexly curved rolling surfaces (13, 15) which bear on one another,
wherein the first jaw part (7a) and the second jaw part (7b) are pivotable with respect to the tool head (5) independently of one another in a tool plane (x-z) which is essentially perpendicular to the bending plane (y-z), and
wherein the first jaw part (7a) and the second jaw part (7b) each define a cylinder-jacket-shaped or cone-jacket-shaped tool control cable surface (29a,b), on which at least one tool control cable section (27a,b) in the form of spiral section can be partly wound on and wound off,
wherein a first rolling surface (13) of the at least two rolling surfaces (13, 15) is divided by way of a web (17) which runs in the bending plane (y-z) into a first section and a second section, and a second rolling surface (15) of the at least two rolling surfaces (13, 15) is divided by way of a groove (19) which runs in the bending plane (y-z) into a first section and a second section, wherein the web (17) at each bending position of the tool head (5) engages at least partly into the groove (19) wherein the web (17) at the apex side defines two bending control cable guides,
wherein the bending control cable guides run symmetrically with respect to the longitudinal axis,
wherein each of the tool control cables (11a,b) comprises a first tool control cable section (23a,b) a second tool control cable section (25a,b) and a third tool control cable section (27a,b), wherein the first tool control cable section (23a,b) leads essentially in the tool plane (x-z) and essentially in a straight line on a first lateral side in the longitudinal direction of the shank (3) from the shank to the respective jaw part (7a,b), wherein the second tool control cable section (25a,b) leads essentially in the tool plane (x-z) essentially in a straight line on a second lateral side which is diametrically opposite the first lateral side, in the longitudinal direction of the shank (3) from the shank (3) to the respective jaw part (7a,7b), and the third tool control cable section (27a,b) connects to the first and the second tool control cable section (25a,b, 27a,b) and is non-positively connected to the respective jaw part (7a,7b), wherein the third tool control cable section (27a) of the first jaw part (7a) is spread out with respect to the third tool control cable section (27b) of the second jaw part (7b), from the shank (3) towards the jaw parts (7a, b) in the bending plane (y-z), wherein the third tool control cable section (27a, b) which runs on the tool control cable surface (29a,b) is led in a lateral outer region of the tool control cable surface (29a,b) into the respective jaw part (7a,b).

2. An instrument (1) according to claim 1, wherein a first (13) of the at least two rolling surfaces (13, 15) are located at a proximal end region of the tool head (5) and a second (15) of the at least two rolling surfaces (13, 15) is located on the distal shank end (2), wherein the first rolling surface (13) at each rolling point with the second rolling surface (15) in the bending plane (y-z) has a greater convex rolling curvature than the second rolling surface (15).

3. An instrument (1) according to claim 2, wherein the at least two rolling surfaces (13, 15) comprise toothings which engage into one another.

4. An instrument (1) according to one of the preceding claims, wherein the web (17) in a central region about the longitudinal axis is higher with respect to the first rolling surface (13) than in the lateral outer regions.

5. An instrument (1) according to one of the preceding claims, wherein the web (17) symmetrically with respect to the longitudinal axis in the bending plane (y-z) at least in sections defines web curvature, wherein the web curvature is smaller than the first rolling curvature.

6. An instrument (1) according to one of the preceding claims, wherein the web (17) defines a central angle bend (20) in the bending plane (y-z) and/or at least in sections the web curvature decreases with the distance to the longitudinal axis.

7. An instrument (1) according to one of the preceding claims, wherein the bending control cable guides comprise a groove (22) for the at least partial insertion of a bending control cable (9).

8. An instrument (1) according to one of the preceding claims, with a bending control cable (9) which runs essentially in the bending plane (y-z) and which comprises a first bending control cable section (9a), a second bending control cable section (9b) and a third bending control cable section (9a), wherein the first bending control cable section (9a) leads in an essentially straight line on a third lateral side in the longitudinal direction of the shank (3) from the shank (3) to the tool head (5), wherein the second bending control cable section (9b) leads essentially in a straight line on a fourth lateral side which is diametrically opposite to the third lateral side, in the longitudinal direction of the shank (3) from the shank (3) to the tool head (5), and the third bending control cable section (9c) connects to the first (9a) and the second bending control cable section (9b) and is non-positively connected to the tool head (5).

9. An instrument (1) according to claim 8, wherein the third bending control cable section (9c) is lead through the tool head (5) in a meandering manner.

10. An instrument (1) according to claim 8 or 9, wherein the third bending control cable section connects the first and the second bending control cable section.

11. An instrument (1) according to claim 8, 9 or 10, wherein the third bending control cable section (9c) defines a bending control cable end of the first (9a) and of the second bending control cable section (9b), wherein the respective bending control cable end is non-positively coupled to the tool head by way of a friction fit, loop guide and/or an end thickening.

12. An instrument (1) according to one of the preceding claims, wherein the tool control cable surface (29a, b) in the tool plane (x-z) defines a diameter (D) and wherein the jaw parts (7a,b) are mounted in a tool receiver (33) of the tool head (5) which runs slot-like manner in the tool plane (x-z), over a common tool axis (8) which runs perpendicularly to the tool plane (x-z), wherein the slot-like tool receiver (33) is deeper in the longitudinal direction than the diameter (D) of the tool control cable surfaces (29a,b).

13. An instrument (1) according to one of the preceding claims, wherein the third tool control cable section (27a,b) is led through the respective jaw part (7a,b) in a meandering manner.

14. An instrument (1) according to one of the preceding claims, wherein the third tool control cable section (27a,b) connects the first (23a,b) and the second tool control cable section (25a,b).

15. An instrument (1) according to one of the preceding claims, wherein the third tool control cable section (27a,b) defines a tool control cable end of the first (23a,b) and second tool control cable section (25a,b), wherein the respective tool control cable end is non-positively coupled to the respective jaw part (7a,b) by way of a friction fit, loop guide and/or an end thickening.

16. An instrument (1) according to one of the preceding claims, wherein the third tool control cable section (27a,b) at least partially is wound in the form of a spiral section on the tool control cable surface (29a,b) of the respective jaw part (7a,b).

17. An instrument (1) according to one of the preceding claims, wherein each jaw part (7a,b) comprises a tool section (31a,b) which extends radially away from the tool control cable surface (29a,b), wherein the third tool control cable section (27a,b) which runs on the tool control cable surface (29a,b), in the circumferential direction is led into the respective jaw part (7a,b) in front of or behind the tool section (31a,b) and is led therein in a loop-like manner.

## Revendications

1. Instrument (1), comprenant
- une tête d'outil (5) articulée sur une extrémité distale (2) d'une tige allongée (3),
- une paire de parties de mâchoire (7a,b) pour cisailler, pincer ou saisir, la paire de parties de mâchoire (7a,b) comportant une première partie de mâchoire (7a) et une seconde partie de mâchoire (7b), et
- un premier câble de commande d'outil (11a) pour la première partie de mâchoire (7a) et un second câble de commande d'outil (11b) pour la seconde partie de mâchoire (7b),
la tête d'outil (5) pouvant être coudée de manière définie par rapport à un axe longitudinal de la tige (3) suivant un angle polaire (θ) dans un plan de coudage (y-z) parallèle à l'axe longitudinal au moyen d'au moins deux surfaces de roulement (13, 15) adjacentes à courbure convexe,
la première partie de mâchoire (7a) et la seconde partie de mâchoire (7b) peuvant pivoter indépendamment l'une de l'autre par rapport à la tête d'outil (5) dans un plan d'outil (x-z) sensiblement perpendiculaire au plan de coudage (y-z), et
la première partie de mâchoire (7a) et la seconde partie de mâchoire (7b) définissant chacune une surface pour câble de commande d'outil (29a,b) sensiblement en forme d'enveloppe cylindrique ou d'enveloppe conique, sur laquelle respectivement au moins une partie de câble de commande d'outil (27a,b) sous la forme d'une portion d'hélice peut être enroulée ou déroulée partiellement,
une première surface de roulement (13) desdites au moins deux surfaces de roulement (13, 15) étant divisée en une première partie et une seconde partie au moyen d'une nervure (17) s'étendant dans le plan de coudage (y-z), et une deuxième surface de roulement (15) desdites au moins deux surfaces de roulement (13, 15) étant divisée en une première partie et une seconde partie au moyen d'une rainure (19) s'étendant dans le plan de coudage (y-z), la nervure (17) s'engageant au moins partiellement dans la rainure (19) pour chaque position de coudage de la tête d'outil (5),
la nervure (17) définissant, côté sommital, deux guides de câble de commande de coudage,
les guides de câble de commande de coudage s'étendant symétriquement par rapport à l'axe longitudinal,
chacun des câbles de commande d'outil (11a,b) comportant une première partie de câble de commande d'outil (23a,b), une deuxième partie de câble de commande d'outil (25a,b) et une troisième partie de câble de commande d'outil (27a,b), la première partie de câble de commande d'outil (23a,b) allant de la tige à la partie de mâchoire (7a,b) respective sensiblement dans le plan d'outil (x-z) et sensiblement en ligne droite sur une première face latérale dans la direction longitudinale de la tige (3), la deuxième partie de câble de commande d'outil (25a,b) allant de la tige (3) à la partie de mâchoire (7a,b) respective sensiblement dans le plan d'outil (x-z) et sensiblement en ligne droite sur une seconde face latérale diamétralement opposée à la première face latérale dans la direction longitudinale de la tige (3), et la troisième partie de câble de commande d'outil (27a,b) se raccordant respectivement à la première et la deuxième partie de câble de commande d'outil (25a,b, 27a,b) et étant en liaison avec transmission de force avec la partie de mâchoire (7a,b) respective,
la troisième partie de câble de commande d'outil (27a) de la première partie de mâchoire (7a) étant expansée, par rapport à la troisième partie de câble de commande d'outil (27b) de la seconde partie de mâchoire (7b), de la tige (3) vers les parties de mâchoires (7a,b) dans le plan de coudage (y-z), la troisième partie de câble de commande d'outil (27a,b), qui s'étend respectivement sur la surface pour câble de commande d'outil (29a,b), étant guidée jusque dans la partie de mâchoire (7a,b) respective dans une région externe latérale de la surface pour câble de commande d'outil (29a,b).

2. Instrument (1) selon la revendication 1, une première (13) desdites au moins deux surfaces de roulement (13, 15) se trouvant à une région d'extrémité proximale de la tête d'outil (5) et une deuxième (15) desdites au moins deux surfaces de roulement (13, 15) se trouvant à l'extrémité de tige distale (2), la première surface de roulement (13) ayant, à chaque point de roulement avec la deuxième surface de roulement (15) dans le plan de coudage (y-z), une courbure de déroulement convexe plus grande que la seconde surface de roulement (15).

3. Instrument (1) selon la revendication 2, lesdites au moins deux surfaces de roulement (13, 15) présentant des dentures entrant mutuellement en prise.

4. Instrument (1) selon l'une des revendications précédentes, la nervure (17), dans une région centrale autour de l'axe longitudinal, étant plus haute par rapport à la première surface de roulement (13) que dans des régions externes latérales.

5. Instrument (1) selon l'une des revendications précédentes, la nervure (17) définissant au moins en partie une courbure de nervure symétriquement par rapport à l'axe longitudinal dans le plan de coudage (y-z), la courbure de nervure étant plus petite que la première courbure de déroulement.

6. Instrument (1) selon l'une des revendications précédentes, la nervure (17) définissant un coude central (20) dans le plan de coudage (y-z) et/ou, au moins en partie, la courbure de nervure diminuant avec la distance par rapport à l'axe longitudinal.

7. Instrument (1) selon l'une des revendications précédentes, les guides de câble de commande de coudage présentant une rainure (22) pour insérer, au moins en partie, un câble de commande de coudage (9).

8. Instrument (1) selon l'une des revendications précédentes, comprenant un câble de commande de coudage (9) s'étendant sensiblement dans le plan de coudage (y-z), qui présente une première partie de câble de commande de coudage (9a), une deuxième partie de câble de commande de coudage (9b) et une troisième partie de câble de commande de coudage (9c), la première partie de câble de commande de coudage (9a) allant de la tige (3) à la tête d'outil (5) sensiblement en ligne droite sur une troisième face latérale dans la direction longitudinale de la tige (3), la deuxième partie de câble de commande de coudage (9b) allant de la tige (3) à la tête d'outil (5) sensiblement en ligne droite sur une quatrième face latérale diamétralement opposée à la troisième face latérale dans la direction longitudinale de la tige (3), et la troisième partie de câble de commande de coudage (9c) se raccordant respectivement à la première (9a) et la deuxième partie de câble de commande de coudage (9b) et étant en liaison avec transmission de force avec la tête d'outil (5),

9. Instrument (1) selon la revendication 8, la troisième partie de câble de commande de coudage (9c) étant guidée en forme de méandre à travers la tête d'outil (5).

10. Instrument (1) selon la revendication 8 ou 9, la troisième partie de câble de commande de coudage reliant la première et la deuxième partie de câble de commande de coudage.

11. Instrument (1) selon la revendication 8, 9 ou 10, la troisième partie de câble de commande de coudage (9c) définissant respectivement une extrémité de câble de commande de coudage de la première (9a) et de la seconde partie de câble de commande de coudage (9b), l'extrémité de câble de commande de coudage respective étant couplée avec transmission de force à la tête d'outil par liaison par frottement, guidage en forme de boucle et/ou par un épaississement terminal.

12. Instrument (1) selon l'une des revendications précédentes, la surface pour câble de commande d'outil (29a,b) définisant un diamètre (D) dans le plan d'outil (x-z) et les parties de mâchoire (7a,b) étant logées dans un logement d'outil (33), en forme de fente, de la tête d'outil (5), s'étendant dans le plan d'outil (x-z), par l'intermédiaire d'un axe d'outil (8) commun s'étendant perpendiculairement au plan d'outil (x-z), le logement d'outil (33) en forme de fente étant, dans la direction longitudinale, plus profond que le diamètre (D) des surfaces pour câble de commande d'outil (29a,b).

13. Instrument (1) selon l'une des revendications précédentes, la troisième partie de câble de commande d'outil (27a,b) étant guidée en forme de méandre à travers la partie de mâchoire (7a,b) respective.

14. Instrument (1) selon l'une des revendications précédentes, la troisième partie de câble de commande d'outil (27a,b) reliant la première (23a,b) et la deuxième partie de câble de commande d'outil (25a,b).

15. Instrument (1) selon l'une des revendications précédentes, la troisième partie de câble de commande d'outil (27a,b) définissant respectivement une extrémité de câble de commande d'outil de la première (23a,b) et de la deuxième partie de câble de commande d'outil (25a,b), l'extrémité de câble de commande d'outil respective étant couplée avec transmission de force à la partie de mâchoire (7a,b) respective par liaison par frottement, guidage en forme de boucle et/ou par un épaississement terminal.

16. Instrument (1) selon l'une des revendications précédentes, la troisième partie de câble de commande d'outil (27a,b) étant enroulée au moins en partie sous la forme d'une portion en hélice sur la surface pour câble de commande d'outil (29a,b) de la partie de mâchoire (7a,b) respective.

17. Instrument (1) selon l'une des revendications précédentes, chaque partie de mâchoire (7a,b) présentant une partie d'outil (31a,b) s'étendant radialement à partir de la surface pour câble de commande d'outil (29a,b), la troisième partie de câble de commande d'outil (27a,b), qui s'étend sur la surface pour câble de commande d'outil (29a,b), étant guidée, dans la direction circonférentielle, devant et derrière la partie d'outil (31a,b), jusque dans la partie de mâchoire (7a,b) respective et dans celle-ci en forme de boucle.
